# EUROPÄISCHE PATENTSCHRIFT

(11) **EP 2 103 220 B1**
(45) Veröffentlichungstag und Bekanntmachung des Hinweises auf die Patenterteilung: **26.09.2018**
(21) Anmeldenummer: 08004971.1
(22) Anmeldetag: 17.03.2008
(51) Int. Cl.: A21D 8/04, A23P 20/20, A23L 29/00, A23L 7/109, A21D 13/16, C12N 9/02

(54) **Verfahren zur Herstellung von laminiertem Teig, Sulfhydryl-Oxidase enthaltend**
Method for manufacturing a laminated dough comprising sulfhydryl oxidase enzyme
Procédé de fabrication de pâte laminée comprenant de l'oxydase sulfhydryle

(43) Veröffentlichungstag der Anmeldung: 23.09.2009
(73) Patentinhaber: Stern Enzym GmbH & Co. KG, 22926 Ahrensburg (DE)
(72) Erfinder: Popper, Lutz, 20257 Hamburg (DE)
(74) Vertreter: Moré, Solveig Helga

(56) Entgegenhaltungen:
- EP-A- 0 565 172
- EP-A- 0 705 538
- WO-A-2005/104856
- US-A- 5 547 690
- US-A- 5 942 262

## Beschreibung

Die vorliegende Erfindung betrifft die Verwendung von Sulfhydryl-Oxidase (SOX), um die Eigenschaften von laminierten Teigen und deren Gebäcken zu verbessern. Die Erfindung betrifft ferner ein Verfahren zum Herstellen von Teig, Teiglingen oder Backwaren, bei dem man SOX mit anderen Zutaten mischt und den Teig laminiert, sowie einen laminierten Teig, laminierten Teigling oder laminierte Backwaren, der/die SOX umfasst/umfassen.

### Stand der Technik

Aus der Literatur (siehe z.B. Wrigley, C, Bekes, F, and Bushuk, W, 2006. Gliadin and glutenin. AACC International) ist bekannt, dass die Struktur des Weizenklebers und somit die rheologischen Eigenschaften des Mehls von Disulfidbrücken zwischen benachbarter Thiolgruppen abhängen. Durch Oxidationsmittel wie Wasserstoffperoxid oder Glucose-Oxidase lassen sich noch freie oder durch natürliche reduzierende Substanzen wie Glutathion frei gewordene Thiolgruppen zu weiteren Disulfidbrücken oxidieren und somit die Teige verfestigen.

Der Anteil von Weizenkleber im Mehl beträgt ca. 10 % der Gesamtmasse. Etwa 2,4 % davon sind Cysteingruppen. Teige weisen einen hohen Feststoffanteil (Mehl) von ca. 60 % auf. Die Proteine liegen in teilhydratisierter, nicht jedoch gelöster Form vor.

Sulfhydryl-Oxidase (SOX) gehört zur Klasse der Oxidoreduktasen (EC 1.8.3.2). Sie katalysiert die Oxidation von zwei Sulfhydrylgruppen zu einer Disulfidbrücke unter Abspaltung von Wasserstoff-Peroxid nach dem Schema 2 RSH + O₂ = RSSR + H₂O₂. Frühere Veröffentlichungen beschreiben die Verwendung von SOX in Kombination mit anderen Enzymen wie Glucose-Oxidase (US 5,547,690*,* EP 0 321 811), Cellulasen oder Hemicellulasen (US 4,990,343*,* EP 0 705 538) als vorteilhaft zur Verbesserung des Backverhaltens von Mehlen.

US 5,942,262 beschreibt die Verwendung einer Proteinase und eines Enzyms, das ein oxidierendes Agens herstellt, z.B. SOX, zur Inaktivierung der Proteinase, wobei die Proteinase zur Hydrolyse von Teig zur Herstellung von Keksen eingesetzt wird. Bei dieser Anwendung dient SOX also zur Kontrolle (im Sinne von Inaktivierung) der Enzymaktivität der Protease. WO 2005/104856 A offenbart eine Backvormischung zur Verbesserung von Backwaren, enthaltend mindestens ein Enzym aus der Gruppe umfassend Amylase, Hemicellulase, Glucose-Oxidase, Amyloglucosidase, Lipase, Phospholipase, Sulfhydryl-Oxidase, Protease, Peroxidase. CA 2 660 863 A1 offenbart eine Backvormischung, enthaltend Roggenmehl, Kleber, Kleberverstärker und Hefe. Der Kleberverstärker enthält wenigstens eine Lipase und/oder wenigstens eine Xylanase und/oder wenigstens eine Hemicellulase und/oder wenigstens eine Oxidase (b.v. SOX) und/oder ein chemisch Oxidant. Eine stabilisierende Wirkung von Sulfhydryl-Oxidase ohne gleichzeitige Verwendung anderer Enzyme in Backprozessen wurde getestet, jedoch nicht gefunden (Kaufman and Fennema, 1986. Evaluation of Sulfhydryl Oxidase as a Strengthening Agent for Wheat Flour Dough Cereal Chem. 64(3), 172-176).

Laminierte Teige sind Teige, die während der Teigzubereitung dünn ausgerollt werden, insbesondere mehrfach dünn ausgerollt, z.B. indem sie ein Rollenpaar passieren. Beispiele für laminierte Teige sind chinesische Dampfbrötchen. Bei deren Herstellung wird die Teigmasse nur kurz gemischt und anschließend durch 30 - 50-maliges Passieren eines Rollenpaares (*dough breaker*) entwickelt. Laminierter Teig wird bevorzugt mit einem *dough breaker* oder vergleichbaren Systemen entwickelt.

Ein weiterer geläufiger Backprozess unter Einbeziehung eines Laminierschrittes ist die Herstellung von Croissants. Insbesondere bei der Herstellung von gefrorenen Croissant-Teiglingen ist eine hohe Stabilität und Gärtoleranz der Teiglinge erforderlich. Bei Butter-haltigen Gebäcken kann diese in geringem Maße durch Ascorbinsäure, einem in Teigen oxidativ wirkenden Mehlbehandlungsmittel erreicht werden. Glucose-Oxidase wird hier als Oxidationsmittel nicht eingesetzt, da es durch die in Gang gesetzten unspezifischen Oxidationsprozesse zu Aroma- und Geschmacksveränderungen bis hin zu Ranzigkeit kommen kann.

Dem gegenüber stellt sich dem Fachmann das Problem, laminierte Teige, Teiglinge und deren Gebäcke mit verbesserten Eigenschaften bzw. Verfahren zu deren Herstellung bereitzustellen.

### Beschreibung der Erfindung

Nun wurde überraschend gefunden, dass Sulfhydryl-Oxidase dann eine starke Wirkung entfaltet, wenn die Teige laminiert, d.h. während der Teigbereitung dünn ausgerollt werden. Der Teig wird sodann gefaltet oder in mehreren Schichten übereinander gelegt, so dass mindestens 2 Lagen und durch wiederholtes Laminieren ein Vielfaches davon entstehen, insbesondere mindestens 4, mindestens 6, mindestens 10 oder mindestens 30 Lagen. Alternativ kann der Teig nach dem Ausrollen auch spiralförmig gewickelt werden, so dass mehrere Schichten aufeinander liegen.

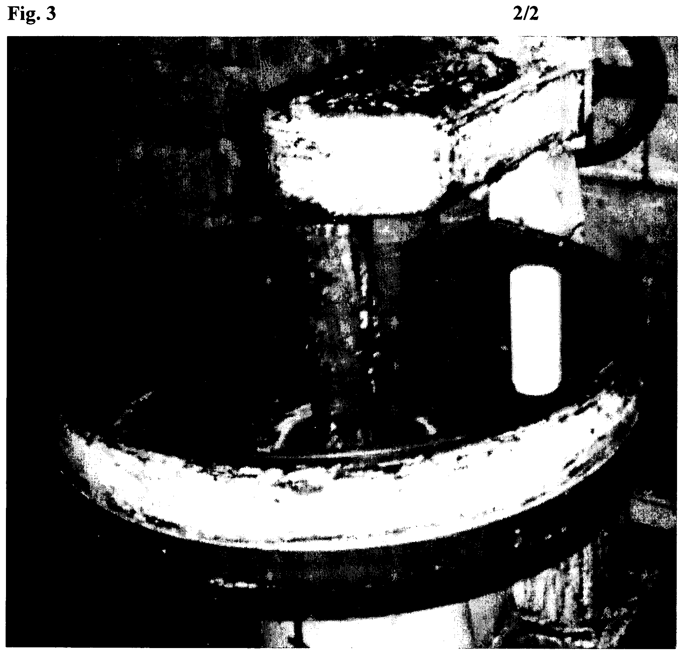

In einer einfachen Ausführung steht ein rotierendes Rollenpaar am inneren Umfang einer rotieren Teigschüssel. Durch die Drehung der Schüssel wird der Teig immer wieder durch das Rollenpaar gezwungen und anschließend an der Wandung zu einem Klumpen aufgerollt, bevor er wieder durch das Rollenpaar gezogen wird (Fig. 3).

Gegenstand der Erfindung ist damit ein Verfahren zum Herstellen von Teig, Teiglingen oder Backwaren, nach Anspruch 1.

Gegenstand der Erfindung ist auch die Verwendung von Sulfhydryl-Oxidase naar Anspruch 8.

Bevorzugt wird der Teig mindestens einmal, noch bevorzugter mehrfach ausgerollt oder laminiert, z.B. zweimal oder häufiger, dreimal oder häufiger, fünfmal oder häufiger, 10-60 mal oder 30-50 mal.

Das Laminieren kann durch Passieren eines Rollenpaares, z.B. mit einem dough breaker (z.B. G. Paniz Indústria de Equipamentos para Alimentação Ltda, Caxias do Sul, Brasil) oder vergleichbaren Systemen erfolgen. Der Abstand zwischen den Rollen, der die Schichtdicke definiert, kann dabei zwischen etwa 1 und etwa 30 mm betragen; dieser Rollenabstand ist von dem herzustellenden Gebäck abhängig. Die erfindungsgemäßen Vorteile ergeben sich sowohl dann, wenn zwischen die Schichten kein Fett eingebracht wird, als auch bei Einbringen von Fett (z.B. Butter), wie etwa bei der Herstellung von Croissants oder Blätterteig.

Die Schritte der Zubereitung des laminierten Teigs, der Teiglinge oder Backwaren können - nach Zusatz der SOX - nach konventionellen Verfahren durchgeführt werden.

Im Rahmen der Erfindung zeigte sich überraschenderweise, dass SOX, nicht aber andere Enzyme wie Glucose-Oxidase (GOX) bei der Herstellung von laminiertem Teig besonders günstige Effekte etwa auf das Backverhalten, Volumenzunahme oder Geschmacks- und Lagerungseigenschaften aufwies. Ohne durch diese Hypothese gebunden sein zu wollen, könnte es sein, dass das Laminieren den Effekt hat, dass die Proteinstränge laminar und parallel ausgerichtet werden. Es könnte sein, dass diese Ausrichtung die Bildung von Disulfidbrücken gerade durch das Enzym SOX begünstigt.

Bevorzugt werden 1-500 milli-Einheiten (mu) SOX/kg Mehl eingesetzt, insbesondere 5-200 mu SOX/kg oder 50-100 mu SOX/kg. Dabei wird auf die in Beispiel 3 beschriebene Methode zur Bestimmung der Aktivität des Enzyms Bezug genommen.

Normalerweise sind weitere Zutaten im Teig Mehl und Wasser, es können z.B. auch Salz, Zucker, Hefe und/oder Backpulver enthalten sein, gegebenenfalls auch Fett (z.B. Butter), Milch und/oder Ei. Als weitere Zutat oder Zutaten können auch ein oder mehrere weitere Mehlbehandlungsmittel ausgewählt aus der Gruppe bestehend aus Enzymen, Reduktions- oder Oxidationsmitteln und Emulgatoren eingesetzt werden, z.B. Ascorbinsäure. Durch die Verwendung von SOX ist es jedoch oft möglich, die standardmäßig verwendete Menge an Reduktions- und Oxidationsmittel zu verringern oder ganz darauf zu verzichten.

Wird SOX zur Herstellung einer Backvormischung verwendet, so umfasst diese bevorzugt bereits Mehl. Optional können auch z.B. Salz, Zucker, Backtriebmittel, Fett, Eipulver, Milchpulver und/oder Emulgatoren bereits enthalten sein. Bevorzugt enthält die Backvormischung noch kein Wasser.

Im Rahmen der vorliegenden Erfindung ist es möglich, aber überraschenderweise nicht notwendig, als weitere Zutaten für Backvormischung, Teig, Teiglinge (bzw. die daraus hergestellten Backwaren) Glucose-Oxidase, Cellulase und/oder Hemicellulase (oder andere Enzyme) einzusetzen. Es wird damit auch ein Verfahren zur Herstellung von Verfahren zum Herstellen von Teig, Teiglingen oder Backwaren bereitgestellt, bei dem man Glucose-Oxidase, Cellulase und/oder Hemicellulase (oder andere Enzyme) nicht als weitere Zutat einsetzt.

Sulfhydryl-Oxidase konnte bislang nur wenig in technischem Maßstab eingesetzt werden, da das Enzym durch den Cofaktor FAD sowie zahlreiche oxidationsempfindliche Cysteingruppen im Enzymmolekül selbst nicht sehr stabil war. Durch den Austausch der Cysteingruppen durch Serin mittel Protein Engineering (DE 103 09 169) wurde die Produktion einer stabileren SOX möglich. Als Basis kann z.B. SOX aus Bierhefe, *Saccharomyces cerevisiae,* verwendet werden. Im Rahmen der vorliegenden Erfindung ist SOX bevorzugt eine gegenüber dem Wildtyp stabilere SOX, insbesondere eine in DE 10309169 beschriebene SOX. In der eingesetzten SOX ist bevorzugt mindestens ein Cysteinrest so verändert, dass die SOX stabiler ist, insbesondere ist mindestens ein redoxaktives Cystein gegen ein Serin ausgetauscht.

SOX ist kommerziell erhältlich von SternEnzym GmbH & Co. KG, Ahrensburg.

Besondere Vorteile ergeben sich erfindungsgemäß, wenn man Teiglinge herstellt, die tiefgefroren werden, z.B. bei Temperaturen von ca. -18°C oder weniger, z.B. ca. -30°C oder Einfrosten bei ca. -30°C, Lagerung bei ca. -18°C. Ein Beispiel sind Teiglinge für Croissants. Dabei umfasst das erfindungsgemäße Verfahren ferner Schritte, bei denen man
c) einen Teigling herstellt
d) den Teigling einfriert.

Insbesondere bei einer längeren Lagerung von erfindungsgemäß hergestellten Teiglingen, z.B. über mehr als eine Woche, mehr als drei Wochen, mehr als zehn Wochen, tritt - anders als bei Einsatz herkömmlicher Backzusätze - hier keine negative Geschmacksveränderung auf.

Mit oder ohne vorheriges Einfrieren kann aus dem Teigling durch Backen die fertige Backware hergestellt werden. Backen umfasst dabei im Zusammenhang mit der vorliegenden Erfindung auch z.B. ein Dämpfen.

Zum Beispiel können erfindungsgemäß vorteilhaft chinesische Dampfbrötchen, Croissants, Blätterteiggebäck, Plundergebäck, Baklava-ähnliches Gebäck (z.B. Baklava), Pasteten, Teig oder Teiglinge zur Herstellung derselben hergestellt werden. Gegenstand der Erfindung ist auch ein laminierter Teig, laminierte Teiglinge oder laminierte Backwaren, welche SOX umfassen. Diese sind wie oben beschrieben herstellbar.

Wie dort erörtert, umfassen die erfindungsgemäßen Teige, Teiglinge oder Backwaren in einer Ausführungsform keine Glucose-Oxidase, Cellulase und/oder Hemicellulase. Diese Enzyme stören jedoch auch nicht oder können u.U. auch positiv mit SOX zusammenwirken.

Insbesondere handelt es sich um laminierten Teig, laminierte Teiglinge oder laminierte Backwaren aus der Gruppe von chinesischen Dampfbrötchen, Croissants, Blätterteig- und Plundergebäck, Baklava-ähnlichem Gebäck (z.B. Baklava), Pasteten, Teig oder Teiglinge zur Herstellung derselben. In einer Ausführungsform handelt es sich um tiefgefrorene Teiglinge, z.B. für Croissants.

Die sehr spezifische Wirkung von SOX auf Sulfhydryl-Gruppen und die damit einhergehende nur sehr geringe Bildung von Wasserstoff-Peroxid (ca. 1/1000 im Vergleich zu Glucose-Oxidase, bezogen auf die rheologische Wirkung) ermöglicht die Stabilisierung der laminierten Teige, Teiglinge und Backwaren ohne negative Geschmacks- und Aromaeffekte. Backversuche zeigten eine deutliche Volumenvergrößerung und Stabilisierung der hergestellten Backwaren.

### Legende

**Fig. 1** **Vergleich der Wirkung von Glucose-Oxidase (GOX) und Sulfhydryl-Oxidase (SOX) auf Volumen und Form von chinesischen Dampfbrötchen.** Als Grundbehandlung des Mehls (Type 550 einer Mischung aus kanadischem CWRS-Weizen und US-amerikanischen DNS-Weizen) wurden 40 ppm Ascorbinsäure eingesetzt (siehe Grundrezeptur). Dem Vergleichmuster (ganz links) wurden 40 ppm Ascorbinsäure zugesetzt, den anderen Versuchsmustern stattdessen GOX bzw. SOX in den angegebenen Konzentrationen (Units per Gramm bzw. milli-Units per Kilogramm).
**Fig. 2** **und** **3** **Dough Breaker**

### Beispiele

### Beipiel 1. Dampfbrötchen

### a) Grundteigzusammensetzung

| **Bestandteil** | **Menge (g)** |
|---|---|
| Mehl (Type 550) | 1000.000 |
| Hefe, Instant | 14.000 |
| Zucker | 125.000 |
| Ascorbinsäure | 0.040 |
| Speisesalz | 13.000 |
| Wasser | 474.920 |

### b) Teigverarbeitung

| | |
|---|---|
| Kneten (Spiralkneter Diosna SP 24) | 6 min langsam, |
| | 3 min schnell |
| Teigruhe | 60 min |

### c) Teigaufarbeitung

| | |
|---|---|
| Teigeinlage (verwendete Teigmenge) | 800 g |
| Laminieren (Seewer-Rondo, Walzenabstand 8 mm) | 40 Durchläufe |
| Langrollen (Baguette Moulder, Walzenabstand 6 mm, Tuchabstand Stufe 2) | 1 Durchlauf |
| Schneiden (manuell, Messer, glatt) | 70 mm |

### d) Gar- und Backprozess

| | |
|---|---|
| Stückgare (35 °C, 82 % rel. F.) | 45 min (Übergare: + 20 min) |
| Dämpfen (Edelstahldämpfer, elektrisch, Stufe 10) | 20 min |

### e) Backergebnisse

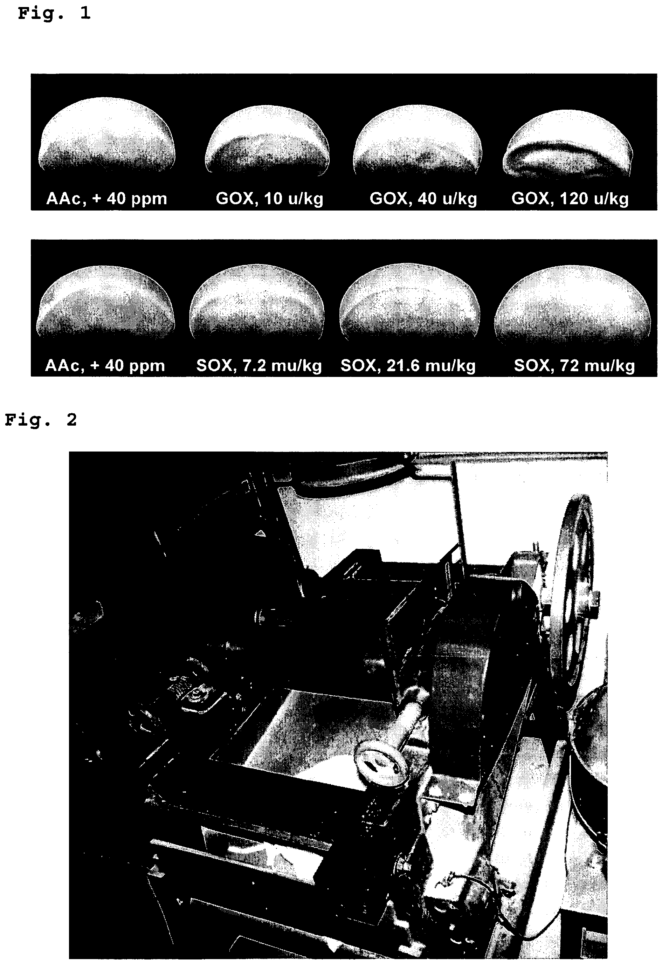

Die Backergebnisse sind in Fig. 1 dargestellt. Insbesondere ist dort die deutlich stärkere Volumenzunahme der mit SOX hergestellten Dampfbrötchen gegenüber GOX und Ascorbinsäure (Aac) sichtbar.

### Beispiel 2. Butter-Croissants aus gefrorenen Teiglingen

Obwohl Croissant-Teige sehr viel weniger laminiert werden als Teige für chinesische Dampfbrote und zudem die Teigschichten durch eine Fettbarrieren (das zwischen den Lagen befindliche Tourierfett, z.B. Butter) voneinander getrennt sind, führt auch hier die Verwendung von SOX zu klaren Vorteilen. Gegenüber dem Standard ohne weitere Backzusätze ist eine klare Volumenzunahme der mit SOX hergestellten Croissants erkennbar. Insbesondere ist im Vergleich zu mit Glucose-Oxidase (GOX) oder auch Ascorbinsäure (Asco) behandelten Teiglingen auch nach einer mehrwöchigen Lagerung keine negative Veränderung von Aroma oder Geschmack festzustellen.

### f) Grundteigzusammensetzung

| **Bestandteil** | **Menge (g)** |
|---|---|
| Mehl (Type 550) | 1500.000 |
| Hefe, frisch | 75.000 |
| Zucker | 120.000 |
| Vollei | 75.000 |
| Vollmilchpulver | 22.500 |
| Ascorbinsäure | 0.120 |
| Speisesalz | 30.000 |
| Wasser | 780.000 |
| Butter zum Tourieren | 750.000 |

### g) Teigverarbeitung

| | |
|---|---|
| Kneten (Spiralkneter Diosna SP 3 min langsam, 3 min schnell 24) | |
| Teigruhe | 60 min |

### h) Teigaufarbeitung

| | |
|---|---|
| Teigeinlage (verwendete Teigmenge) | 400 g |
| Touren (Laminieren und Einarbeitung von Butter; (Seewer-Rondo, Walzenabstand 3.5 mm) | 3 x 3 |
| Teigruhe (2 °C, 82 % rel. F.) | 120 min |
| Touren (wie oben) | 1 x 3 |
| Schneiden (Länge x Breite) | 20 x 10 cm (Dreiecke) |

### i) Gefrierlagerung

| | |
|---|---|
| Einfrosten (MiWe Gärunterbrecher) | - 30 °C, 1 d |
| Lagern (26 bzw. 70 d) | - 18 °C |

### j) Gar- und Backprozess

| | |
|---|---|
| Stückgare (27 °C, 75 % rel. F.) | 80 min |
| Backen (MiWe Aeromat, 175 °C) | 20 min |

### k) Backergebnisse

Backvolumina (in mL pro Stück) und sensorische Auffälligkeiten nach direktem Backen bzw. nach Lagerung von 26 bzw. 70 Tagen bei -18 °C.

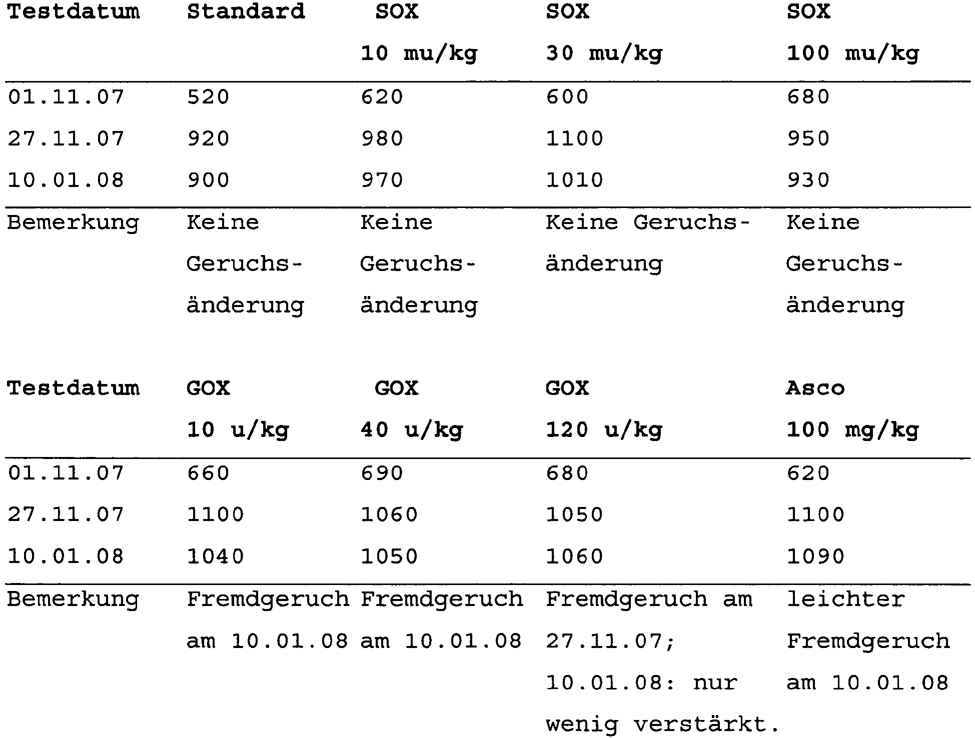

### Beispiel 3. Sulfhydryl-Oxidase Aktivitätstest

Enzymatische Aktivität wird durch Überwachen der Abnahme von freien Thiol-Gruppen in reduziertem DTT bestimmt. Unter Verwendung von DTNB [5,5'-dithiol-bis(2-nitrobenzoylsäure)] wird der Thiolgehalt vor und nach Hinzufügen des Enzyms spektroskopisch bei 412 nm bestimmt (E_{DNTP} = 13.6 mM⁻¹ * cm⁻¹).

Eine Einheit(u) Aktivität wird als die Menge Enzym definiert, die mit 1 mM DTT als Substrat bei pH 7,4 und 30 °C in mit Phosphat gepufferter Salzlösung (PBS) 1 µmol freier Thiolgruppen pro Minute umsetzt.

### Test

DTT (10 mM) in 100 µl PBS (68 mM NaCl, 75 mM K-Phosphat-Puffer pH 7,4) wird 800 µl PBS + 100 µl DTNB (4 mg/10 ml PBS)hinzugefügt, wobei eine Konzentration von DTT ausgewählt wird, die nach Hinzufügen von DTNB zu einer OD₄₁₂ von 0.6 - 0.8 führt.

Dazu wird Enzym (50 pmol FAD gebunden) + DTT in 100 µl PBS gegeben. Die Mischung wird für 5, 10, 15, 20 oder 25 min bei 30 °C inkubiert. Die Reaktion wird durch Zugabe von 800 µl PBS + 100 µl DTNB gestoppt und die OD₄₁₂ nach 30 sec gemessen.

### Bestimmung der spezifischen Enzymaktivität

E_{reference} - Eₜₑₛₜ/13.6 · min⁻¹ · mg⁻¹ · Volumen der Küvette = nmol Thiol-Gruppen, die pro mg Enzym und min oxidiert wurden.

## Patentansprüche

1. Verfahren zum Herstellen von Teig, Teiglingen oder Backwaren, Schritte umfassend, bei denen man
a) einen Teig aus Sulfhydryl-Oxidase (SOX) und weiteren Zutaten herstellt, wobei der Teig Hefe umfasst, und
b) den Teig laminiert
wobei Teig, Teigling oder Backware durch SOX stabilisiert werden, wobei Teig oder Teigling zur Herstellung einer Backware verwendet werden, und das Volumen der Backware erhöht wird.

2. Verfahren zum Herstellen von Teiglingen oder Backwaren nach Anspruch 1, ferner Schritte umfassend, bei denen man
c) einen Teigling herstellt,
d) den Teigling einfriert.

3. Verfahren nach einem der vorherigen Ansprüche, **dadurch gekennzeichnet, dass** man den Teig mehrfach laminiert.

4. Verfahren nach einem der vorherigen Ansprüche, **dadurch gekennzeichnet, dass** SOX eine gegenüber dem Wildtyp stabilere SOX ist.

5. Verfahren nach einem der vorherigen Ansprüche, **dadurch gekennzeichnet, dass** man Backwaren ausgewählt aus der Gruppe umfassend chinesische Dampfbrötchen, Croissants, Blätterteiggebäck, Plundergebäck, Baklava-ähnliches Gebäck, Pasteten, und Teig oder Teiglinge zur Herstellung derselben herstellt.

6. Verfahren nach einem der vorherigen Ansprüche, **dadurch gekennzeichnet, dass** als weitere Zutat oder Zutaten auch ein oder mehrere weitere Mehlbehandlungsmittel ausgewählt aus der Gruppe umfassend Enzyme, Reduktions- oder Oxidationsmittel oder Emulgatoren eingesetzt werden.

7. Verfahren nach einem der Ansprüche 1 bis 5, **dadurch gekennzeichnet, dass** keine weiteren Enzyme eingesetzt werden.

8. Verwendung von Sulfhydryl-Oxidase (SOX) zur Stabilisierung von laminiertem Teig, laminierten Teiglingen oder laminierten Backwaren oder zur Herstellung einer Backvormischung zur Herstellung von laminiertem Teig, wobei der Teig Hefe umfasst, wobei Teig oder Teigling zur Herstellung einer Backware verwendet werden, wobei das Volumen der Backware erhöht wird.

9. Verwendung nach Anspruch 8, **dadurch gekennzeichnet, dass** der Teig, der Teigling oder die Backware tiefgekühlt wird.

10. Verwendung nach einem der Ansprüche 8 oder 9, **dadurch gekennzeichnet, dass** der Teig mehrfach laminiert ist.

11. Verwendung nach einem der Ansprüche 7-10, **dadurch gekennzeichnet, dass** SOX eine gegenüber dem Wildtyp stabilere SOX ist.

12. Verwendung nach einem der Ansprüche 7-11, **dadurch gekennzeichnet, dass** die Sulfhydryl-Oxidase zur Herstellung von Backwaren ausgewählt aus einer Gruppe umfassend chinesische Dampfbrötchen, Croissants, Blätterteiggebäck, Plundergebäck, Baklava-ähnliches Gebäck, Pasteten, Teig, Teiglingen oder einer Backvormischung zur Herstellung dieser Backwaren verwendet wird.

13. Verwendung nach einem der Ansprüche 7-12, **dadurch gekennzeichnet, dass** zusätzlich ein oder mehrere weitere Mehlbehandlungsmittel ausgewählt aus der Gruppe bestehend aus Enzymen, Reduktions- oder Oxidationsmitteln oder Emulgatoren eingesetzt werden.

14. Verwendung nach einem der Ansprüche 7-12, **dadurch gekennzeichnet, dass** keine weiteren Enzyme eingesetzt werden.

15. Laminierter Teig, laminierte Teiglinge oder laminierte Backwaren, welche SOX umfassen, wobei der Teig Hefe umfasst.

16. Laminierter Teig, laminierte Teiglinge oder laminierte Backwaren nach Anspruch 15, herstellbar nach einem der Ansprüche 1-7.

17. Laminierter Teig, laminierte Teiglinge oder laminierte Backwaren nach Anspruch 15 oder 16, **dadurch gekennzeichnet, dass** keine weiteren Enzyme enthalten sind.

## Claims

1. A method for manufacturing dough, dough pieces or bakery products comprising steps in which one
a) manufactures a dough from sulfhydryl oxidase enzyme (SOX) and further ingredients, wherein the dough comprises yeast, and
b) laminates the dough
wherein dough, dough piece or bakery product are stabilized by SOX, wherein dough or dough piece are used for manufacturing a bakery product, and the volume of the bakery product is increased.

2. The method for manufacturing dough pieces or bakery products according to claim 1, further comprising steps in which one
c) manufactures a dough piece,
d) freezes the dough piece.

3. The method according to any of the preceding claims, **characterized in that** one laminates the dough repeatedly.

4. The method according to any of the preceding claims, **characterized in that** SOX is a more stable SOX compared to the wild type.

5. The method according to any of the preceding claims, **characterized in that** one manufactures bakery products selected from the group comprising Chinese steamed buns, croissants, puff pastries, Danish pastries, Baklava-like pastries, pasties, and manufactures dough or dough pieces for manufacturing the same.

6. The method according to any of the preceding claims, **characterized in that** one or more further flour treatment agents selected from the group comprising enzymes, reduction or oxidation agents or emulsifying agents are also applied as further ingredient or ingredients.

7. The method according to any of claims 1 to 5, **characterized in that** no further enzymes are applied.

8. Use of sulfhydryl oxidase enzyme (SOX) for stabilisation of laminated dough, laminated dough pieces or laminated bakery products or for manufacturing a baking premix for manufacturing a laminated dough, wherein the dough comprises yeast, wherein dough or dough piece are used for manufacturing a bakery product, wherein the volume of the bakery product is increased.

9. The use according to claim 8, **characterized in that** the dough, the dough piece or the bakery product is deep-frozen.

10. The use according to any of claims 8 or 9, **characterized in that** the dough is laminated repeatedly.

11. The use according to any of claims 7-10, **characterized in that** SOX is a more stable SOX compared to the wild type.

12. The use according to any of claims 7-11, **characterized in that** the sulfhydryl oxidase enzyme is used for manufacturing bakery products selected from a group comprising Chinese steamed buns, croissants, puff pastries, Danish pastries, Baklava-like pastries, pasties, dough, dough pieces or a baking premix for manufacturing said bakery products.

13. The use according to any of claims 7-12, **characterized in that** one or more further flour treatment agents selected from the group consisting of enzymes, reduction or oxidation agents or emulsifying agents are additionally applied.

14. The use according to any of claims 7-12, **characterized in that** no further enzymes are applied.

15. A laminated dough, laminated dough pieces or laminated bakery products, which comprise SOX, wherein the dough comprises yeast.

16. The laminated dough, laminated dough pieces or laminated bakery products according to claim 15, manufacturable according to any of claims 1-7.

17. The laminated dough, laminated dough pieces or laminated bakery products according to claim 15 or 16, **characterized in that** no further enzymes are contained.

## Revendications

1. Procédé pour la production de pâte, de pâtons ou de produits de boulangerie, comprenant les étapes dans lesquelles
a) on prépare une pâte à partir de sulfhydryl-oxydase (SOX) et d'autres ingrédients, la pâte comprenant de la levure, et
b) on soumet la pâte à un laminage
la pâte, le pâton ou le produit de boulangerie étant stabilisés par la SOX, la pâte ou le pâton étant utilisés pour la fabrication d'un produit de boulangerie, et le volume du produit de boulangerie étant augmenté.

2. Procédé pour la production de pâtons ou de produits de boulangerie selon la revendication 1, comprenant encore les étapes dans lesquelles
c) on prépare un pâton,
d) on congèle le pâton.

3. Procédé selon l'une quelconque des revendications précédentes, **caractérisé en ce qu'**on soumet plusieurs fois la pâte à un laminage.

4. Procédé selon l'une quelconque des revendications précédentes, **caractérisé en ce que** la SOX est une SOX plus stable que le type sauvage.

5. Procédé selon l'une quelconque des revendications précédentes, **caractérisé en ce qu'**on choisit les produits de boulangerie dans l'ensemble comprenant les petits pains chinois cuits à la vapeur, les croissants, les pâtisseries à pâte feuilletée, les viennoiseries, les pâtisseries de type baklava, les pâtés en croûte, et la pâte ou les pâtons pour la fabrication de tels produits.

6. Procédé selon l'une quelconque des revendications précédentes, **caractérisé en ce que** comme autre(s) ingrédient ou ingrédients on utilise également un ou plusieurs autre(s) agent(s) de traitement de la farine choisi(s) dans l'ensemble comprenant les enzymes, les oxydants ou réducteurs ou les émulsifiants.

7. Procédé selon l'une quelconque des revendications 1 à 5, **caractérisé en ce qu'**on n'utilise pas d'autres enzymes.

8. Utilisation de sulfhydryl-oxydase (SOX) pour la stabilisation de pâte laminée, de pâtons laminés ou de produits de boulangerie laminés ou pour la production d'un prémélange de boulangerie destiné à la production de pâte laminée, la pâte comprenant de la levure, la pâte ou le pâton étant utilisés pour la fabrication d'un produit de boulangerie, le volume du produit de boulangerie étant augmenté.

9. Utilisation selon la revendication 8, **caractérisée en ce que** la pâte, le pâton ou le produit de boulangerie est surgelé(e).

10. Utilisation selon l'une quelconque des revendications 8 et 9, **caractérisée en ce que** la pâte est laminée plusieurs fois.

11. Utilisation selon l'une quelconque des revendications 7 à 10, **caractérisée en ce que** la SOX est une SOX plus stable que le type sauvage.

12. Utilisation selon l'une quelconque des revendications 7 à 11, **caractérisée en ce qu'**on utilise la sulfhydryl-oxydase pour la fabrication de produits de boulangerie choisis dans un ensemble comprenant les petits pains chinois cuits à la vapeur, les croissants, les pâtisseries à pâte feuilletée, les viennoiseries, les pâtisseries de type baklava, les pâtés en croûte, la pâte, les pâtons ou un prémélange de boulangerie destiné à la fabrication de tels produits.

13. Utilisation selon l'une quelconque des revendications 7 à 12, **caractérisée en ce qu'**on utilise en outre un ou plusieurs autres(s) agent(s) de traitement de la farine choisi(s) dans l'ensemble constitué par les enzymes, les oxydants ou réducteurs ou les émulsifiants.

14. Utilisation selon l'une quelconque des revendications 7 à 12, **caractérisée en ce qu'**on n'utilise pas d'autres enzymes.

15. Pâte laminée, pâtons laminés ou produits de boulangerie laminés, qui comprennent de la SOX, la pâte comprenant de la levure.

16. Pâte laminée, pâtons laminés ou produits de boulangerie laminés selon la revendication 15, pouvant être produits selon l'une quelconque des revendications 1 à 7.

17. Pâte laminée, pâtons laminés ou produits de boulangerie laminés selon la revendication 15 ou 16, **caractérisés en ce que** d'autres enzymes ne sont pas contenues.
